# EUROPEAN PATENT APPLICATION

(11) **EP 3 977 887 A1**
(43) Date of publication of application: **06.04.2022**
(21) Application number: 21199025.4
(22) Date of filing: 27.09.2021
(51) Int. Cl.: A43B 7/28, A43B 17/00, A61F 5/14

(54) **METHOD FOR PRODUCING A SOLE STIFFENER FOR AN ORTHOPAEDIC SHOE, SOLE STIFFENER AND AN ORTHOPAEDIC SHOE PROVIDED WITH A SOLE STIFFENER**

(30) Priority: 30.09.2020 NL 2026587
(71) Applicant: CYS Group B.V., 6093 PL Heythuysen (NL)
(72) Inventor: DE VOS, Antonius Wilhelmus, HEYTHUYSEN (NL)
(74) Representative: Algemeen Octrooi- en Merkenbureau B.V.

(57) **Abstract**

The invention relates to a method for producing a sole stiffener for an orthopaedic shoe. The sole stiffener is provided with an insole from which a middle section is or has been removed so that the insole is formed by a border. Carbon fibre material in combination with a bonding material is applied in an inner space formed by the removed middle section together with the border to obtain an insole reinforced with carbon fibre material.

## Description

The present invention is aimed at a method for producing a sole stiffener for an orthopaedic shoe. The present invention is further aimed at a sole stiffener. Lastly, the invention is aimed at an orthopaedic shoe comprising a sole stiffener.

In one aspect, an object of the present invention is to provide a new method for producing an improved sole stiffener for an orthopaedic shoe. In another aspect, an object is to provide a method for producing an improved sole stiffener for an orthopaedic shoe in an operator-safe manner.

At least one of these objects is achieved by means of the method for producing a sole stiffener for an orthopaedic shoe as defined in Claim 1.

In the method for producing a sole stiffener for an orthopaedic shoe, the sole stiffener is provided with an insole from which a middle section is or has been removed so that the insole is formed by a border, wherein carbon fibre material in combination with a bonding material is applied in an inner space formed by the removed middle section together with the border to obtain an insole reinforced with carbon fibre material.

The insole is made of a material, for example a plastic, that is easy for an operator to work and presents no or practically no health risk for the operator during the production of the insole. Thus, a middle section may be removed from the insole in a relatively straightforward and operator-safe manner. After removal of the middle section, all that remains is an (endless) border of the insole. By virtue of the chosen workable material of the insole, the insole, in the form of the border, can be detachably fastened to temporary aids by an operator in a relatively straightforward manner for the one or more subsequent operations to be performed by the operator, in particular for, for example, applying carbon fibre material in combination with a bonding material in an inner space formed by the removed middle section together with the border in order to obtain an insole reinforced with carbon fibre material. The bonding material provides bonding of the carbon fibre material to the border, among other things. Despite the carbon fibre material provided therein, an insole produced in this way can be made relatively thin. For example, the insole can be roughly 3 mm thick, which corresponds to the thickness of a standard insole, so that the insole reinforced with carbon fibre material produced using the method can be integrated into an orthopaedic shoe and into the process for producing an orthopaedic shoe in a relatively straightforward manner without consequences with respect to a standard insole. The sole stiffener obtained using the method can therefore be implemented in a straightforward manner in the production of an orthopaedic shoe without requiring adjustments in the process for producing the orthopaedic shoe. An important advantage of the method mentioned above is that after applying the carbon fibre material in combination with the bonding material, it is not necessary to further manually process or grind/sand the carbon fibre material, which processes on carbon fibre material can be particularly harmful for the health of an operator. Additionally, the relatively straightforward method requires only simple basic training for the operator. The sole stiffener produced by means of the method is also particularly lightweight and can, for example, be fastened via the border in a manner known to an operator using, for example, tacks, screws or staples, or other known detachable fastening means, for example to the temporary aids mentioned above or ultimately to the orthopaedic shoe. If such detachable fastening means are used to fasten the sole stiffener to the shoe, where the detachable fastening means are not made to be manually detachable by an end user, it is even possible in particular (emergency) cases for an operator to remove the sole stiffener from the orthopaedic shoe using aids, for example a tool. What is meant by manually detachable in this document is that a person (operator or end user) can separate components from one another by hand, whereas detachably fastened means that the components cannot normally be separated from one another by hand and aids are required. Furthermore, the insole can be made so as to be practically unbendable, relatively strong, and practically unbreakable using the method, as a result of which it is particularly applicable as an anti-penetration insole for orthopaedic safety shoes.

When removing, for example by cutting out, the middle section in the case of an insole not yet made to measure for the end user, a relatively safe distance, for example of a few centimetres (more than 2 cm) from the outer periphery of the insole should be retained. When removing the middle section in the case of an insole already made to measure for the end user, a smaller distance with respect to the outer periphery of the insole can be used, for example a distance of between 0.5 and 2 cm, such as 1 cm.

The insole can, before or after the application of the carbon fibre material in combination with the bonding material, be made to measure for the end user with the aid of an orthotic made to measure for the end user together with a last made to measure for the end user. For that, the insole can be detachably fastened to the orthotic together with the last, after which the insole can be made to measure for the end user in a straightforward and operator-safe manner by removing material from an outer edge of the insole so that the insole has an outer periphery that corresponds to the outer periphery of the orthotic. Optionally, a space sole made or to be made to measure for the end user can be arranged between the insole and the orthotic so that the insole, the orthotic and the space sole are fastened to the last, after which the insole, and possibly the space sole, is or are made to measure for the end user. Such space soles are known per se, for example in order to achieve a desired or end user-prescribed thickness in the final orthopaedic shoe. In the process for producing the sole stiffener, it is normally advantageous, in the light of subsequent operations to be performed, to make the insole to measure for the end user in the manner described above before applying the carbon fibre material in combination with the bonding material.

For the inner space of the insole, strips made of carbon fibre material can be used, which strips are provided, or impregnated, for example by an operator, with bonding material before the strips are applied in the inner space. The inner space of the insole is completely or at least substantially filled with the strips of carbon fibre material in combination with the bonding material. The bonding material bonds the strips together and bonds the strips to the inner edge of the insole in order to obtain an integral unit. In one aspect, the strips of carbon fibre material to be used in the method are formed by stocking-like strips of carbon fibre material. Furthermore, the bonding material can, for example, be a mixture of polyester resin and hardener.

Particularly advantageously when producing a sole stiffener, but also when producing the orthopaedic shoe, the insole reinforced with carbon fibre material is detachably connected to other objects such as the orthotic, the last and/or the space sole and even to the orthopaedic shoe via the border using the fastening means described above. The border may be an endless border, which increases the handleability of the border for the operator.

The invention further relates to a sole stiffener produced using the method set out above. The sole stiffener for an orthopaedic shoe as claimed is provided with an insole made to measure for the end user with a border that defines an inner space in which carbon fibre material in combination with a bonding material is applied to obtain an insole reinforced with carbon fibre material. The advantages of such a sole stiffener have already been mentioned above.

The inner space of the insole provided substantially with carbon fibre material in combination with a bonding material can form at least 50% of the total volume of the insole, preferably at least 65%. Producing an insole of the sole stiffener in this way provides a practically unbendable sole stiffener which is particularly strong and cannot or practically cannot break in an orthopaedic shoe. For an operator to process the sole stiffener, it is possible to detachably fasten the insole via the border to an orthotic made to measure for the end user. Optionally, there is a space sole made to measure for the end user therebetween. For comfort and/or for aesthetic reasons, at least one side of the insole reinforced with carbon fibre material can further be covered with a covering layer, for example made of Alcantara, so that, with the aid of the covering layer on the at least one side, preferably at least the upper side, the carbon fibre material of the insole is not visually perceptible.

Lastly, the invention relates to an orthopaedic shoe comprising a sole stiffener as described above. In the orthopaedic shoe, an orthotic can be placed on the insole reinforced with carbon fibre material of the orthopaedic shoe in a manually detachable manner. A space sole made to measure for the end user can be positioned in the orthopaedic shoe between the orthotic and the insole. Particularly advantageously, the border of the insole can be connected to the orthopaedic shoe in a manner that is not manually detachable, so that the insole reinforced with carbon fibre material can form a part of the orthopaedic shoe that is securely fastened to the orthopaedic shoe.

The above-described aspects together with other aspects of the sole stiffener or the method for producing a sole stiffener will be explained below by means of one exemplary embodiment in combination with the figures. However, the invention is not limited to the exemplary embodiment described below. Rather, a number of variants and modifications are possible which likewise use the inventive idea claimed in the claims and thus fall within the scope of protection. In particular, the possibility of combining properties/aspects which have only been mentioned in the description and/or illustrated in the figures with the features of the claims insofar as compatible is mentioned.
Figure 1 shows perspective views of parts that are usable in a method for making a sole stiffener;
Figure 2 shows various steps in obtaining an insole reinforced with carbon fibre material;
Figure 3 shows a perspective view of a pressing machine with the aid of which an insole reinforced with carbon fibre material can be produced;
Figure 4 shows various possible layers to be applied in an orthopaedic shoe in addition to the improved sole stiffener in order to achieve the desired orthopaedic support for an end user.

In the figures, identical parts are provided with the same reference signs.

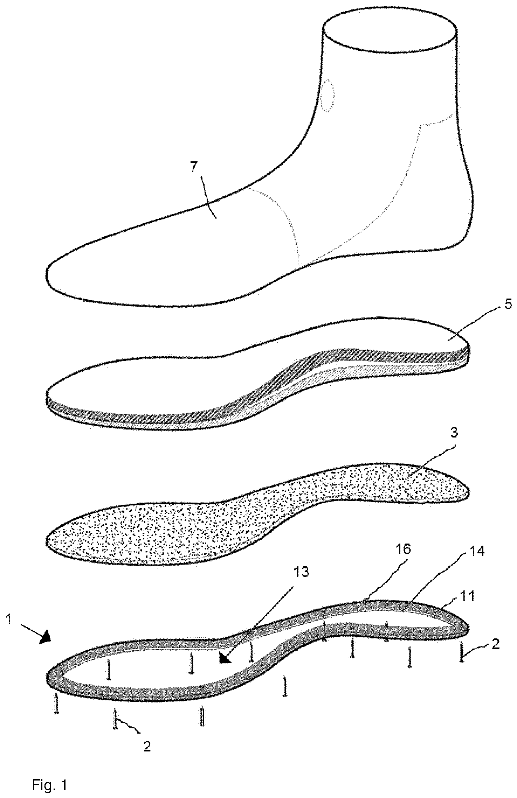

Figure 1 shows an insole 1 that is to be fastened to a space sole 3, to an orthotic 5 and to a last 7 using nails 2.

The last 7 is an individual last for an end user which is made, for example from wood, on the basis of precise measurements and scans of an end user's foot in a known manner.

The orthotic 5 is likewise individually made to measure for the end user in a known manner. Orthotics are also known as orthopaedic footbeds, inlays, correction soles, inlay soles or supplements. The function of an orthotic 5 is normally to correct the state of an end user's foot.

Optionally, a space sole 3 made or to be made to measure for the end user can be arranged between the insole 1 and the orthotic 5 so that, as shown in Figure 1, the insole 1, the orthotic 5 and the space sole 3 can be fastened to the last 7. Such space soles are known per se, for example in order to achieve a desired or end user-prescribed thickness in the orthopaedic shoe.

In the method for producing a sole stiffener described below, the last 7 and the orthotic are already an orthotic 5 and last 7 formed according to the individual foot measurements of an end user. The optional space sole 3 can be made to measure for the end user during the method described below or can be a space sole 3 already made to measure for the end user.

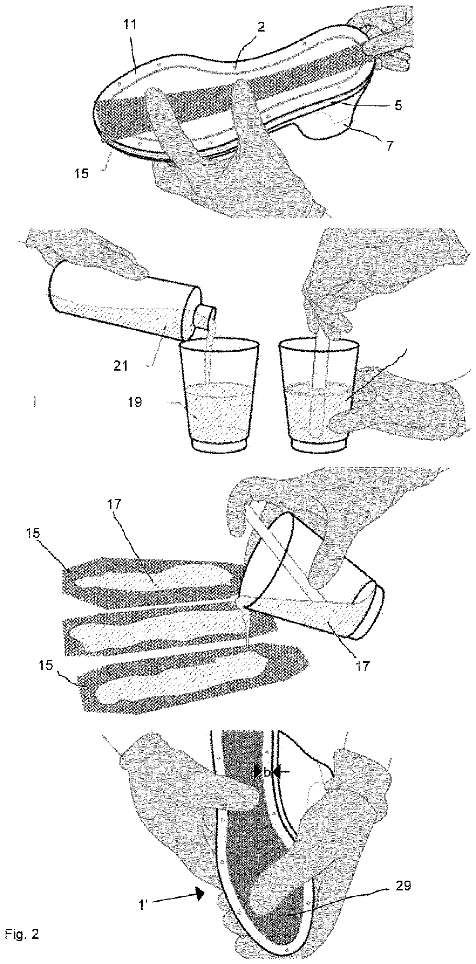

The exemplary embodiment in Figure 2 shows how the method described below can be carried out, in which it can be seen in the steps shown in Figure 2 that in each case the last 7, the orthotic 5 and the insole 1 shown in Figure 1 are detachably fastened to one another using nails 2. Instead of nails 2, other detachable fastening means can also be used. Before the step shown in Figure 2 is carried out, a protective layer, for example a plastic film, is secured around the last 7 and the orthotic 5, and possibly the space sole 3.

In the method for producing a sole stiffener for an orthopaedic shoe (not shown), the sole stiffener is provided with an insole 1 from which a middle section (not shown) is removed, as shown in Figure 1, or can be removed, so that the insole 1 is formed by an endless border 11. Figure 2 shows that a carbon fibre material 15 in combination with a bonding material 17 is applied in an inner space 13 formed by the removed middle section together with the border to obtain an insole 1' reinforced with carbon fibre material.

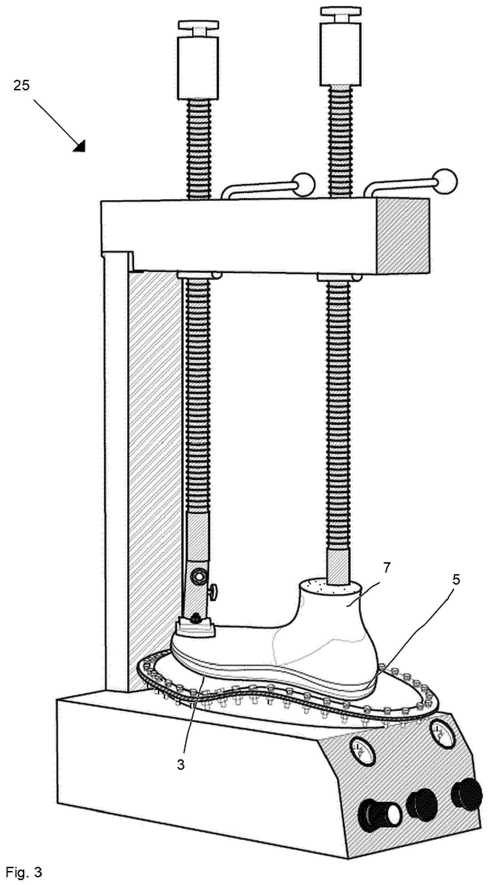

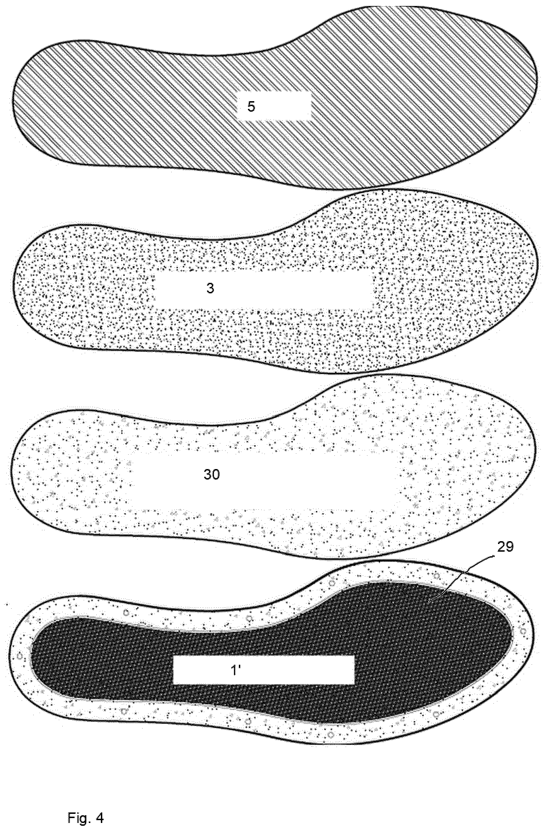

By way of merely illustrative example, the method can comprise the following steps, in which the optional space sole 3 is omitted and some steps are optional:
Step 1 Using a made-to-measure last 7, a standard insole (not shown) is fastened to the orthotic 5 with nails 2.
Step 2 The standard insole fastened to the orthotic 5 and last 7 is processed by removing material from the outer edge of the standard insole so that the insole has an outer periphery that exactly corresponds to the outer periphery of the orthotic layer. The standard insole is now an insole made to measure for the end user (not shown).
Step 3 The made-to-measure insole is removed from the orthotic and the last by an operator by removing the nails 2.
Step 4 A middle section is removed a determined distance from the outer periphery of the made-to-measure insole, for example by an operator or an at least partially mechanical operation, resulting in only an endless insole border 11 of the insole 1 shown in Figure 1 remaining. The distance between inner periphery 14 and the outer periphery 16 of the endless insole border 11 is, for example, 1 cm.
Step 5 The endless insole border 11 is fastened to the orthotic 5 and the last 7 using nails 2.
Step 6 Strips 15, more particularly stocking-like strips 15, are cut to measure, preferably mechanically, from carbon fibre material for subsequent placement in the inner space 13 inside the insole border 11 if no matching stocking-like strips 15 in the shape of the inner space 13 are available (see, for example, the topmost drawing of Figure 2).
Step 7 A bonding means 17 is formed and applied (third drawing from the top in Figure 2), for example by dabbing (not shown), to the upper side and underside of the stocking-like strips 15 so that the stocking-like strips 15 are soaked or impregnated with the bonding means 17. The second drawing of Figure 2 shows how a suitable bonding means 17 can be prepared using a polyester resin 19 and a hardener 21.
Step 8 The stocking-like strips 15 produced in step 7 are applied inside the endless insole border in the inner space 13 of the insole 1' to be produced.
Step 9 The insole 1' is placed in a pressing machine 25 as shown, for example, in Figure 3 for a determined time period for pressing and hardening of the bonding material 17 in order to obtain an insole 1' reinforced with carbon fibre material of the desired thickness.
Step 10 Removing the nails 2, resulting in the insole 1' reinforced with glass fibre material coming free from the last 7 and the orthotic 5.
Step 11 At least a side of the insole 1' reinforced with carbon fibre material that will ultimately face upwards in an orthopaedic shoe is covered with a covering layer 30, as shown in Figure 4; for example, the covering layer 30 is made of Alcantara, so that with the aid of the covering layer, at least the carbon fibre material with the bonding material, denoted in Figures 3 and 4 by the reference numeral 29, is not visually perceptible. The covering layer 30 can, for example, be glued or can be stretched and nailed or stapled to the insole 1'. In the underside of the insole 1' shown in Figure 4, the covering layer 30 is arranged on the upper side (not shown) of the insole 1', whereby the endless border 11 is used for fastening the covering layer 30 such that this is also visible on the underside; for example the covering layer 30 can be nailed to the endless border. Therefore, in Figure 4, the endless border 11 on the underside of the insole 1' is covered with the covering layer, which is represented visually in Figure 4 by using the same shading by which the covering layer 30 is represented in Figure 4.
Step 12 The components shown in Figure 4, namely the orthotic 5, a space sole 3 and the insole 1' reinforced with glass fibre material, the upper side of which is covered with the covering layer 30, are detachably connected using nails (not shown).

After step 11, in a process for producing the orthopaedic shoe (not shown), the insole 1' is detached from the orthotic 5 and the space sole 3.

In the process for producing the orthopaedic shoe, the endless border of the insole is connected to the orthopaedic shoe in a manner that is not manually detachable, so that the insole reinforced with carbon fibre material forms a part of the orthopaedic shoe that is securely fastened to the orthopaedic shoe. In the orthopaedic shoe ultimately produced, the orthotic 5, and possibly a space sole 3 made to measure for the end user, are placed on the insole 1' reinforced with carbon fibre material of the orthopaedic shoe in a manually detachable manner.

In the insole 1' shown, the volume of the inner space of the insole provided substantially with carbon fibre material in combination with a bonding material is between 70% and 80% of the total volume of the insole 1'. To obtain an unbendable and relatively strong sole stiffener with the insole described in this document, the volume of the inner space of the insole provided substantially with carbon fibre material in combination with a bonding material should form at least 50% of the total volume of the insole, preferably at least 65% of the total volume of the insole 1'.

Although not shown in the figures, it is possible, after the operations shown in Figure 2 or 3, to make the insole to measure for the end user by removing material from the outer edge of the insole so that the insole has, in a later stage than in the steps described here, an outer periphery that corresponds to the outer periphery of the orthotic 5. When removing the middle section in the case of an insole not yet made to measure for the end user, a relatively safe distance, for example of a few centimetres (more than 2 cm) from the outer periphery of the insole should be retained, so that after the insole is made to measure, a sufficiently wide endless border 11 of about 1 cm is obtained, given that no detachable fastening means such as nails, screws, tacks or the like can be applied in the inner space filled with carbon fibre material and bonding material to provide the temporary attachments in the production process described above or to provide a connection between the insole 1' and the orthopaedic shoe. The insole is made of a material to which an operator can apply nails, screws, tacks or the like in a relatively straightforward and safe manner in order to produce a detachable connection between the insole and another component in a straightforward manner. The endless border 11 in the made-to-measure insole 1' has a width b of between 0.5 and 2 cm, normally about 1 cm.

Furthermore, while making the insole 1, 1' to measure for the end user, the space sole 3 can also be made to measure in the same process, if the space sole 3 has not already been made to measure.

Instead of the endless border 11 shown, it is possible to produce at least one (minimal) break in the border where the border is provided with ends.

## Claims

1. Method for producing a sole stiffener for an orthopaedic shoe, wherein the sole stiffener is provided with an insole from which a middle section is or has been removed so that the insole is formed by a border, wherein carbon fibre material in combination with a bonding material is applied in an inner space formed by the removed middle section together with the border to obtain an insole reinforced with carbon fibre material.

2. Method according to Claim 1, wherein the insole, before or after the application of the carbon fibre material in combination with the bonding material, is made to measure for the end user with the aid of an orthotic made to measure for the end user together with a last made to measure for the end user.

3. Method according to Claim 2, wherein the insole is detachably fastened to the orthotic together with the last, after which the insole is made to measure for the end user by removing material from an outer edge of the insole so that the insole has an outer periphery that corresponds to the outer periphery of the orthotic, and optionally a space sole made or to be made to measure for the end user is to be arranged between the insole and the orthotic so that the insole, the orthotic and the space sole are fastened to the last, after which the insole, and possibly the space sole, is or are made to measure for the end user.

4. Method according to one of the preceding claims, wherein strips made of carbon fibre material provided, or impregnated, with bonding material are applied in the inner space of the insole, so that the inner space of the insole is at least substantially filled with carbon fibre material in combination with the bonding material, and preferably the strips of carbon fibre material are formed by stocking-like strips of carbon fibre material and/or the bonding material is a mixture of polyester resin and hardener.

5. Method according to one of the preceding claims, wherein the insole reinforced with carbon fibre material is pressed using a pressing machine for a predetermined time period.

6. Method according to one of the preceding claims, wherein at least one side of the insole reinforced with carbon fibre material is covered with a covering layer, for example made of Alcantara, so that, with the aid of the covering layer on the at least one side, preferably the upper side, the carbon fibre material is not visually perceptible.

7. Method according to one of the preceding claims, wherein the insole reinforced with carbon fibre material is detachably connected to the orthotic via the border of the insole, and optionally a space sole made to measure for the end user can be arranged between the insole and the orthotic.

8. Method according to one of the preceding claims, wherein the border is an endless border.

9. Sole stiffener for an orthopaedic shoe, wherein the sole stiffener is provided with an insole made to measure for the end user with a border that defines an inner space in which carbon fibre material in combination with a bonding material is applied to obtain an insole reinforced with carbon fibre material.

10. Sole stiffener according to Claim 9, wherein the inner space of the insole provided substantially with carbon fibre material in combination with a bonding material forms at least 50% of the total volume of the insole, preferably at least 65%, and/or the border is an endless border.

11. Sole stiffener according to Claim 9 or 10, wherein the insole is to be detachably fastened to an orthotic made to measure for the end user via the border, and optionally a space sole made to measure for the end user is to be detachably fastened between an orthotic and the insole reinforced with carbon fibre material.

12. Sole stiffener according to one of Claims 9 to 11, wherein at least one side of the insole reinforced with carbon fibre material is covered with a covering layer, for example made of Alcantara, so that, with the aid of the covering layer on the at least one side, preferably the upper side, the carbon fibre material of the insole is not visually perceptible.

13. Orthopaedic shoe comprising a sole stiffener according to at least one of Claims 9 to 12.

14. Orthopaedic shoe according to Claim 13, wherein, in the orthopaedic shoe, an orthotic is placed on the insole reinforced with carbon fibre material of the orthopaedic shoe in a manually detachable manner, and optionally a space sole made to measure for the end user is to be positioned in the orthopaedic shoe between the orthotic and the insole.

15. Orthopaedic shoe according to one of Claims 13 and 14, wherein the border of the insole is connected to the orthopaedic shoe in a manner that is not manually detachable, so that the insole reinforced with carbon fibre material forms a part of the orthopaedic shoe that is securely fastened to the orthopaedic shoe.
